(19) **Europäisches Patentamt**

**European Patent Office**

**Office européen des brevets**

(11) Veröffentlichungsnummer: **0 083 720**

**A2**

(12) **EUROPÄISCHE PATENTANMELDUNG**

(21) Anmeldenummer: **82110867.7**

(22) Anmeldetag: **24.11.82**

(51) Int. Cl.³: **A 61 M 5/14**

(30) Priorität: **13.01.82 DE 3200724**

(43) Veröffentlichungstag der Anmeldung:
**20.07.83 Patentblatt 83/29**

(84) Benannte Vertragsstaaten:
**AT BE CH DE FR GB IT LI LU NL SE**

(71) Anmelder: **INTERMEDICAT GMBH.**
**Gerliswilstrasse 74**
**CH-6020 Emmenbrücke(CH)**

(72) Erfinder: **Lesemann, Egon**
**Röhnstrasse 3**
**D-3501 Körle(DE)**

(72) Erfinder: **Herlitze, Gerhard**
**Binsdörferstrasse 4**
**D-3507 Baunatal(DE)**

(74) Vertreter: **von Kreisler, Alek, Dipl.-Chem. et al,**
**Deichmannhaus am Hauptbahnhof**
**D-5000 Köln 1(DE)**

(54) Spülvorrichtung für einen Katheter.

㉗ Die Spülvorrichtung weist ein zylindrisches Gehäuse (20) auf, in dem sich ein zylindrischer Ventilkörper (25) befindet. Der Ventilkörper enthält ein querlaufendes Kapillarrohr (30) aus Glas, das in einer ersten Drehstellung des Ventilkörpers zu dem Einlaßkanal (22) und dem Auslaßkanal (23) ausrichtbar ist. In einer anderen Drehstellung des Ventilkörpers (25) sind die Enden eines Umgehungskanals (32,33) zu dem Einlaßkanal (22) und dem Auslaßkanal (23) ausgerichtet. Es besteht die Möglichkeit, in einen an den Auslaßkanal (23) angeschlossenen Katheter durch das Kapillarrohr (30) eine Spülflüssigkeit mit geringer Strömungsrate zu leiten oder eine Schnellspülung oder normale Infusion durchzuführen, bei der der Umgehungskanal (32,33), der eine größere Weite hat, in den Flüssigkeitsweg geschaltet ist.

FIG. 2

EP 0 083 720 A2

## Spülvorrichtung für einen Katheter

Die Erfindung betrifft eine Spülvorrichtung für einen Katheter, mit einem in einen Flüssigkeitsweg einschaltbaren Gehäuse, das ein Kapillarrohr zur Drosselung und Dosierung des Flüssigkeitsstromes und einen das Kapillarrohr umgehenden Umgehungskanal aufweist.

Bei der Katheterisierung von Patienten besteht die Gefahr, daß an dem im Patienten liegenden Katheterende durch Koagulation des Blutes Verstopfungen auftreten, so daß der Durchgang durch den Katheter behindert wird. Die Ursache für solche Verstopfungen kann darin bestehen, daß durch einen vorübergehenden Unterdruck im Katheter Blut oder andere Körperflüssigkeiten in das Katheterende eingesaugt werden. Die Gefahr der Blutgerinnung und des Zusetzens besteht insbesondere bei englumigen Kathetern.

Zum Freihalten des Katheters und zur Verhinderung von Blutgerinnungen am Katheterende ist es bekannt, den Katheter an eine Vorrichtung anzuschließen, die eine stetige geringe Flüssigkeitsströmung durch den Katheter zum Patienten aufrechterhält (DE-PS 21 46 588). Die bekannte Vorrichtung weist ein Gehäuse auf, in dem sich ein die Flüssigkeitsströmung drosselndes und dosierendes Kapillarrohr befindet, das ständig im Flüssigkeitsweg liegt. Bevor der Katheter in den Körper des Patienten eingeführt wird, ist es erforderlich, den Katheter und den Zuleitungsschlauch zum Katheter zu entlüften. Da diese Entlüftung mit der von dem Kapillarrohr durchgelassenen Flüssigkeitsmenge eine sehr lange Zeit erfordern würde, ist parallel zu dem Kapillarrohr ein Umgehungskanal vorgesehen, in dem sich ein Ventil befindet. Dieses Ventil kann durch Ziehen an einem Strang von Hand geöffnet werden. Da der Umgehungskanal einen wesentlich größeren Durchmesser hat als das Kapillarrohr, wird bei geöffnetem Ventil eine erheblich größere Flüssigkeitsmenge pro Zeiteinheit zum Katheter gebracht. Bei der bekannten Vorrichtung ist das Auslaßende des Kapillarrohres mit einem Anschlußstutzen verbunden, an den eine Druckmeßeinrichtung angeschlossen werden kann, die auf Druckimpulse anspricht. Die bekannte Vorrichtung hat verschiedene Nachteile. Ihre Konstruktion ist aufwendig und kompliziert und ihre Herstellung verursacht relativ hohe Kosten. Ein Absperrventil ist an dieser Vorrichtung nicht vorgesehen, so daß mindestens der durch das Kapillarrohr hindurchgehende Flüssigkeitsstrom ständig fließt. Zum Absperren muß ein separates externes Ventil eingesetzt werden.

Ausgehend von dem genannten Stand der Technik liegt der Erfindung die Aufgabe zugrunde, eine Spülvorrichtung zu schaffen, die einfacher im Aufbau und billiger in der Herstellung ist.

- 3 -

Zur Lösung dieser Aufgabe ist erfindungsgemäß vorgesehen, daß das Kapillarrohr und der Umgehungskanal an einem in dem Gehäuse verstellbaren Ventilkörper vorgesehen und derart angeordnet sind, daß in einer ersten Stellung des Ventilkörpers nur das Kapillarrohr und in einer zweiten Stellung des Ventilkörpers nur der Umgehungskanal in dem Flüssigkeitsweg liegt.

Die erfindungsgemäße Spülvorrichtung enthält somit das Kapillarrohr und den Umgehungskanal in einem gemeinsamen Ventilkörper, der in dem Gehäuse verstellbar ist, so daß der Flüssigkeitsdurchgang entweder nur durch das Kapillarrohr oder nur durch den Umgehungskanal hindurch erfolgt. Dies führt zu einer einfachen ventilähnlichen Konstruktion, bei der das Gehäuse als einstückiges Teil zu fertigen ist. Außer dem Gehäuse wird nur noch der Ventilkörper mit dem eingesetzten Kapillarrohr benötigt. Das Gehäuse und der Ventilkörper können als Spritzgußteile aus Kunststoff hergestellt werden.

Der Durchmesser des Umgehungskanals ist wesentlich größer als derjenige des Kapillarrohres, so daß zum Schnellspülen und zum Entlüften des Katheters und des Zuleitungsschlauchs zum Katheter der Umgehungskanal in den Flüssigkeitsweg gestellt werden kann, während das Kapillarrohr aus dem Flüssigkeitsweg herausbewegt ist, d.h. nicht durchströmt wird.

Das Ventil kann entweder als Drehventil, also mit einem in dem Gehäuse drehbaren Küken, ausgebildet sein oder als Ventilschieber, bei dem der Ventilkörper innerhalb des Gehäuses eine Längsbewegung ausführt. Die erste Möglichkeit bietet jedoch den Vorteil einer einfacheren und genaueren Einstellung des Ventilkörpers durch Drehung eines mit dem Ventilkörper verbundenen und aus dem Gehäuse herausragenden Handgriffs.

Gemäß einer bevorzugten Ausführungsform der Erfindung sperrt der Ventilkörper den Flüssigkeitsweg in einer dritten Stellung ab. Damit kann die Spülvorrichtung ohne jeglichen zusätzlichen Aufwand gleichzeitig als Absperrorgan mitbenutzt werden, so daß sich ein zusätzliches externes Absperrventil erübrigt.

In vorteilhafter Weiterbildung der Erfindung ist vorgesehen, daß der Ventilkörper aus einem im wesentlichen zylindrischen Küken besteht, dessen Umfangswand abdichtend an der Innenwand des Gehäuses anliegt und das zwei unter verschiedenen Winkeln zueinander verlaufende Querkanäle aufweist, deren Enden abwechselnd sowohl zu einem Einlaßkanal als auch zu einem Auslaßkanal des Gehäuses ausrichtbar sind. Hierbei werden die verschiedenen Durchfluß- bzw. Absperrzustände durch Drehen des Ventilkörpers eingestellt. Dabei wird in der ersten Stellung das Kapillarrohr zu dem Einlaßkanal und zu dem Auslaßkanal ausgerichtet, so daß der Durchfluß ausschließlich durch das Kapillarrohr erfolgt. In der zweiten Drehstellung werden die Enden des Umgehungskanals zu dem Einlaßkanal und dem Auslaßkanal ausgerichtet, so daß der Durchfluß ausschließlich über den Umgehungskanal erfolgt, während das Kapillarrohr abgesperrt ist. In einer dritten Drehstellung des Ventilkörpers wird der Einlaßkanal und/oder der Auslaßkanal von dem Ventilkörper abgesperrt.

Vorzugsweise verlaufen die Querkanäle im wesentlichen rechtwinklig zueinander. Der das Kapillarrohr enthaltende Querkanal kann geradlinig ausgebildet sein, während der den Umgehungskanal bildende Querkanal an seinen Enden zu dem Ventilkörper achsparallele Nuten aufweist. Auf diese Weise wird erreicht, daß das Kapillarrohr in eine geradlinige Bohrung eingesetzt werden kann, so daß die das Kapillarrohr durchströmende Flüssigkeit von dem Einlaßkanal zum Auslaßkanal geradlinig durchfließt, während in der zweiten Drehstellung des Ventilkörpers die Flüssigkeit in dem Umgehungskanal schleifenförmig umgelenkt wird.

Der den Umgehungskanal bildende Querkanal kann entweder .eine Radialnut in der Stirnwand des Ventilkörpers oder eine durch den Ventilkörper hindurchgehende Bohrung aufweisen.

Im Folgenden werden unter Bezugnahme auf die Zeichnungen zwei.Ausführungsbeispiele der Erfindung näher erläutert.

Es zeigen:

Fig. 1    den Anschluß der Spülvorrichtung an zwei
          Flüssigkeitsbehälter und an den Katheter,

Fig. 2    einen Längsschnitt durch die Spülvorrichtung,

Fig. 3    einen Querschnitt entlang der Linie III-III der
          Fig. 2,

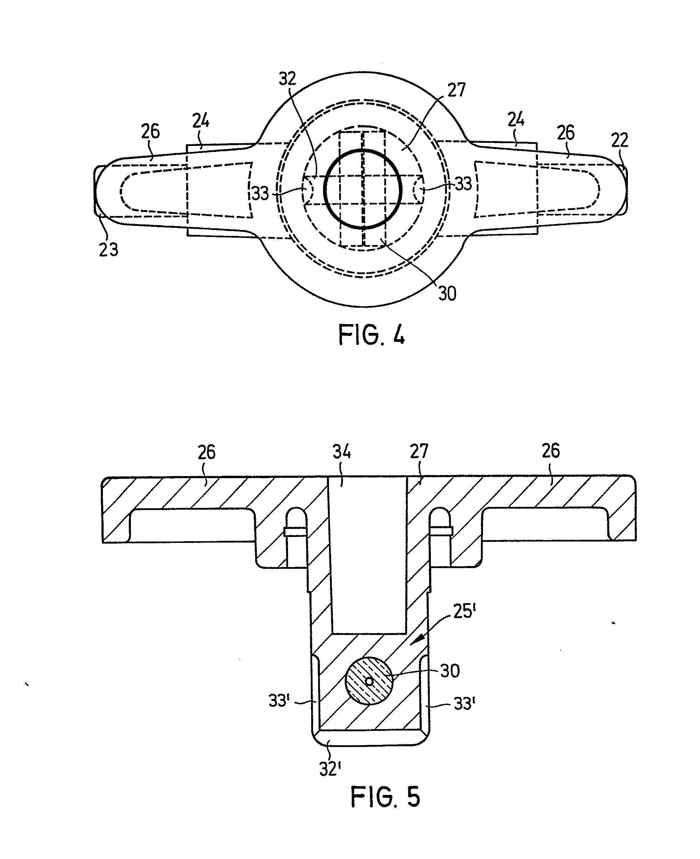

Fig. 4    eine Draufsicht der Spülvorrichtung nach Fig.
          2 und 3, und

- 6 -

Fig. 5      eine zweite Ausführungsform des Ventilkörpers.

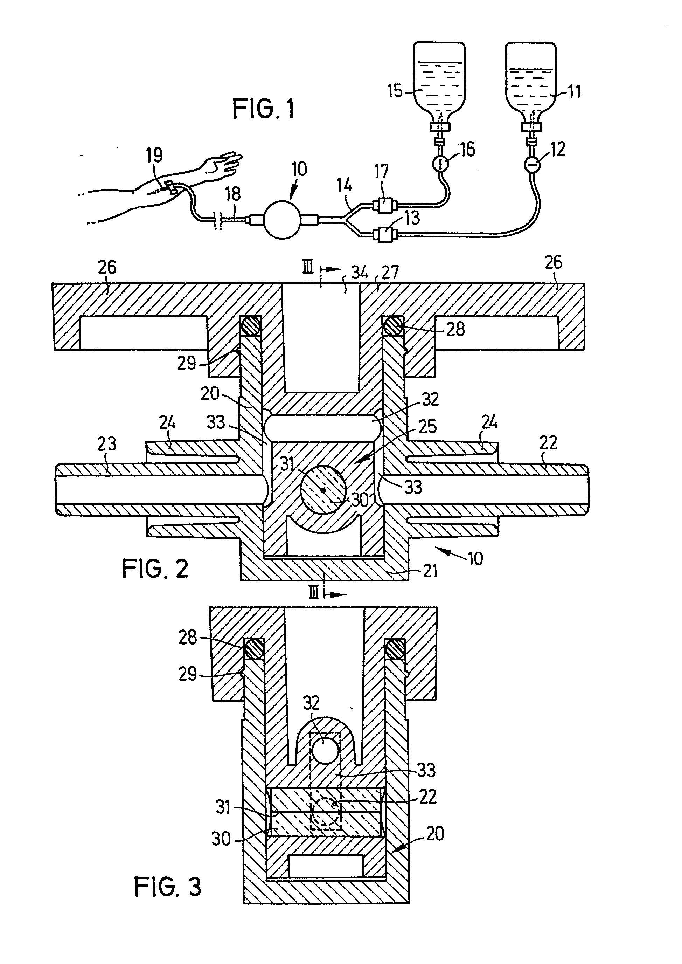

Gemäß Fig. 1 ist eine Flasche 11, die eine Infusionslösung enthält,über ein Absperrventil 12 und ein
Mikronfilter 13 an den einen Einlaßarm eines Y-Stückes
14 angeschlossen. Eine zweite Flasche 15, die eine
Spüllösung, z.B. eine Kochsalzlösung, enthält, ist über
ein Absperrventeil 16 und ein Mikronfilter 17 mit dem
zweiten Einlaßkanal des Y-Stückes 14 verbunden.Der Auslaßkanal des Y-Stückes 14 ist an den Einlaßkanal der
Spülvorrichtung 10 angeschlossen, die nachfolgend noch
erläutert wird. Der Auslaßkanal der Spülvorrichtung 10
ist über einen Anschlußschlauch 18 mit dem patientenfernen Ende eines Katheters 19 verbunden, der in einer
Blutbahn eines Patienten verlegt ist.

Wenn die Infusionslösung aus der Flasche 11 dem Patienten
nicht zugeführt wird und das Absperrventil 12 demnach
geschlossen ist, ist das Absperrventil 16 geöffnet, um die
Spülflüssigkeit in einem konstanten, jedoch sehr geringen
Mengenstrom durch den Katheter 19 hindurchzuleiten.

Die Spülvorrichtung 10 nach den Fig. 2 bis 4 weist ein
Gehäuse 20 mit einer zylindrischen Umfangswand auf, das
nach oben hin offen und nach unten durch eine Bodenwand
21 geschlossen ist. Von der Umfangswand 20 stehen seitlich nach entgegengesetzten Richtungen der Einlaßkanal
22 und der Auslaßkanal 23 ab. Diese Kanäle münden in den
zylindrischen Innenraum des Gehäuses 10. Der Einlaßkanal
22 und der Auslaßkanal 23 sind Rohrstücke, die jeweils
auf einem Teil ihrer Länge von einem koaxialen Schutzmantel 24 mit radialem Abstand umgeben sind. Die Schutzmäntel 24 haben konische Innenflächen und zwischen
ihnen und dem jeweiligen Kanal 22 bzw. 23 kann ein Anschlußschlauch festgeklemmt werden. Natürlich sind
auch andere Anschlußverbindungen, z.B. durch Schlauchkupplungen, möglich.

In das Innere des Gehäuses 10 ragt von oben her der
Ventilkörper 25 hinein. Das obere Ende des Ventilkörpers 25 ist mit zwei radial nach entgegengesetzten
Richtungen abstehenden Handgriffen 26 versehen, die über
die Außenkontur des Gehäuses 10 hinaus seitlich abstehen. Zwischen dem oberen Ende der Seitenwand 20 des
Gehäuses 10 und einer die Handgriffe 26 tragenden
Scheibe 27 befindet sich ein Dichtungsring 28, der in
einer sich nach unten öffnenden Axialnut der Scheibe 27
untergebracht ist. Die Außenwand der Axialnut weist eine
umlaufende Ringnut auf, in der ein Wulst 29 an der Außenseite des Gehäuses 20 einrastet, so daß der Ventilkörper
25 unverlierbar, jedoch drehbar an dem Gehäuse 20 befestigt ist.

Der Außendurchmesser des im wesentlichen zylindrischen
Ventilkörpers 25 entspricht dem Innendurchmesser des
Gehäuses 20. In einer Querbohrung des Ventilkörpers
25 befindet sich das Kapillarrohr 30, bei dem es sich
um einen geraden Glasstab mit einer axialen Bohrung 31
von geringem Durchmesser handelt. Der Durchmesser der
Bohrung 31 beträgt einige hundertstel Millimeter. Die
Bohrung 31 befindet sich auf gleicher Höhe wie die
Längsachsen des Einlaßkanals 22 und des Auslaßkanals 23,
so daß die Bohrung 31 durch Drehen des Ventilkörpers
25 in Ausrichtung zu dem Einlaßkanal 22 und dem Auslaßkanal 23 gebracht werden kann. In diesem Fall fließt die
Flüssigkeit durch die Bohrung des Kapillarrohrs 30 hin-

- 8 -

durch.

Rechtwinklig zu dem Kapillarrohr 31 verläuft eine weitere Querbohrung 32 durch den Ventilkörper 25 hindurch. Die Bohrung 32 ist, bezogen auf die Drehachse des Ventilkörpers 25, relativ zu dem Kapillarrohr 30 versetzt. Sie liegt im vorliegenden Fall oberhalb des Kapillarrohres 30. Von den Enden der Querbohrung 32 erstrecken sich Axialnuten 33 in der Außenwand des Ventilkörpers 25 bis in die Höhe des Einlaßkanals 22 und des Auslaßkanals 23. Die Querbohrung 32 bildet zusammen mit den Längsnuten 33 den Umgehungskanal. Die Weite des Umgehungskanals 32,33 ist an allen Stellen viel größer als die Weite der Bohrung 31 des Kapillarrohrs 30.

Der Durchmesser der Bohrung 31 des Kapilarrohres 30 ist beispielsweise so bemessen, daß bei einem bestimmten Vordruck von 400 mbar die Durchflußrate 2,3,4,5 + 6 cm³/h beträgt.

In der in den Fig. 2 bis 4 gezeigten Stellung des Ventilkörpers 25 erfolgt ein freier Flüssigkeitsdurchgang durch die Vorrichtung. Dieser Zustand dient zum Entlüften des Schlauches 18 und des Katheters 19, sowie zum Schnellspülen.

Zum gedrosselten Spülen wird der Ventilkörper 25 um 90° gedreht, so daß seine Axialnuten 33 aus dem Bereich der Einlaßleitung 22 und der Auslaßleitung 23 herausgedreht werden und die Enden des Kapillarrohres 33 den Enden des Einlaßkanales 22 und des Auslaßkanales 23 gegenüber liegen.

0083720

- 9 -

Zum vollständigen Absperren des Flüssigkeitsstromes wird der Ventilkörper 25 gegenüber der in den Fig. 2 bis 4 dargestellten Position um 45° verdreht.

Die Länge des Kapillarrohres 30 ist etwas kleiner als der Durchmesser des Ventilkörpers 25 (Fig. 3), damit die Ränder der ebenen Stirnseiten des zylindrischen Kapillarrohres 30 die Drehung in dem zylindrischen Gehäuse 20 nicht behindern.

In der dargestellten Position des Ventilkörpers 25, also bei freiem Durchgang und um 90° verschwenktem Kapillarrohr 30, kann eine normale Infusion mit der in der Flasche 11 enthaltenen Infusionslösung durchgeführt werden, wobei das Absperrventil 16 geschlossen und das Absperrventil 12 geöffnet ist.

Der Ventilkörper 25', der in Fig. 5 dargestellt ist, gleicht weitgehend demjenigen der Fig. 2 bis 4, so daß nachfolgend nur die Unterschiede gegenüber dem ersten Ausführungsbeispiel erläutert werden. Der Umgehungskanal besteht bei dem zweiten Ausführungsbeispiel aus einer Radialnut 32', die quer zum Kapillarrohr 30 in der unteren Stirnseite des Ventilkörpers 25' verläuft. Von den Enden der Radialnut 32' führen Axialnuten 33' bis in die Höhe des Einlaßkanals 22 und des Auslaßkanals 23. Das Ausführungsbeispiel von Fig. 5 hat gegenüber dem ersten Ausführungsbeispiel den Vorteil, daß der Materialbedarf für den Ventilkörper 25' noch geringer ist. Dabei ist zu berücksichtigen, daß beide Ventilkörper 25 und 25' einen axialen, nach oben offenen Hohlraum 34 aufweisen, der sich bis in die Nähe der obersten Querbohrung nach unten erstreckt.

0083720

- 10 -

<u>A n s p r ü c h e</u>

1. Spülvorrichtung für einen Katheter, mit einem in einen Flüssigkeitsweg einschaltbaren Gehäuse (20), das ein Kapillarrohr (30) zur Drosselung und Dosierung des Flüssigkeitsstromes und einen das Kapillarrohr (30) umgehenden Umgehungskanal (32,33) aufweist, d a d u r c h g e k e n n z e i c h n e t , daß das Kapillarrohr (30) und der Umgehungskanal (32,33) an einem in dem Gehäuse (20) verstellbaren Ventilkörper (25) vorgesehen und derart angeordnet sind, daß in einer ersten Stellung des Ventilkörpers (25) nur das Kapillarrohr (30) und in einer zweiten Stellung des Ventilkörpers (25) nur der Umgehungskanal (32,33) in dem Flüssigkeitsweg liegt.

2. Spülvorrichtung nach Anspruch 1, dadurch gekennzeichnet, daß der Ventilkörper (25) den Flüssigkeitsweg in einer dritten Stellung absperrt.

3. Spülvorrichtung nach Anspruch 1 oder 2, dadurch gekennzeichnet, daß der Ventilkörper (25) aus einem im wesentlichen zylindrischen Küken besteht, dessen Umfangswand abdichtend an der Innenwand des Gehäuses (20) anliegt und das zwei unter verschiedenen Winkeln zueinander verlaufende Querkanäle aufweist, deren Enden abwechselnd sowohl zu einem Einlaßkanal (22) als auch zu einem Auslaßkanal (23) des Gehäuses (20) ausrichtbar sind.

4. Spülvorrichtung nach Anspruch 3, dadurch gekennzeichnet, daß die Querkanäle im wesentlichen rechtwinklig zueinander verlaufen.

5. Spülvorrichtung nach Anspruch 3 oder 4, dadurch gekennzeichnet, daß der das Kapillarrohr (30) enthaltende
Querkanal geradlinig ausgebildet ist, während der den
Umgehungskanal (32,33) bildende Querkanal an seinen
Enden zu dem Ventilkörper achsparallele Nuten (33) aufweist.

6. Spülvorrichtung nach einem der Ansprüche 3 bis 5, dadurch gekennzeichnet, daß der den Umgehungskanal (32,33)
bildende Querkanal eine Querbohrung (32) aufweist.

7. Spülvorrichtung nach einem der Ansprüche 3 bis 5, dadurch gekennzeichnet, daß der den Umgehungskanal (32',
33') bildende Querkanal eine Radialnut (32') in der
Stirnwand des Ventilkörpers (25') aufweist.

0083720

FIG. 1

FIG. 2

FIG. 3

32    27
26    24                    24    26    22

33            33

23

30

FIG. 4

26        34        27        26

25'

30

33'            33'

32'

FIG. 5